# FASCICULE DE BREVET EUROPEEN

(11) **EP 0 915 857 B1**
(45) Date de publication et mention de la délivrance du brevet: **17.07.2002**
(21) Numéro de dépôt: 97933721.9
(22) Date de dépôt: 11.07.1997
(51) Int. Cl.: C07D 263/44, A61K 7/06, A61K 31/42

(54) **COMPOSES DE LA FAMILLE DES 3-ARYL 2,4 DIOXO OXAZOLIDINES ET LEUR UTILISATION EN COSMETIQUE ET EN PHARMACIE**
VERBINDUNGEN AUS DER GRUPPE DER 3-ARYL-2,4-DIOXO-OXAZOLIDINE UND IHRE VERWENDUNG IN DER KOSMETIK UND PHARMAZIE
COMPOUNDS OF THE 3-ARYL 2,4 DIOXO OXAZOLIDINE FAMILY AND USE THEREOF IN COSMETICS AND PHARMACEUTICALS

(30) Priorité: 01.08.1996 FR 9609748
(43) Date de publication de la demande: 19.05.1999
(73) Titulaire: L'OREAL, 75008 Paris (FR)
(72) Inventeur: GALEY, Jean-Baptiste, F-93600 Aulnay-sous-Bois (FR); BRETON, Lionel, F-78000 Versailles (FR); DESTREE, Odile, F-77270 Villeparisis (FR)
(74) Mandataire: Tezier Herman, Béatrice
(86) Numéro de dépôt international: FR9701289
(87) Numéro de publication internationale: WO9805654

(56) Documents cités:
- DE-A- 3 115 650
- FR-A- 1 284 516
- FR-A- 2 275 206

## Description

La présente invention concerne des composés de la famille des aryl 2,4 dioxo oxazolidines.
Elle concerne également, leur procédé de synthèse, les compositions les contenant ainsi que l'utilisation d'au moins un de ces composés pour induire et/ou stimuler la croissance des cheveux et/ou freiner leur chute et/ou à traiter l'hyperséborrhée et/ou l'acné.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte importante, temporaire ou définitive, des cheveux. L'alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment les golfes temporaux ou frontaux chez l'homme, et chez les femmes, on constate une alopécie diffuse du vertex.

Le terme alopécie recouvre toute une famille d'atteintes du follicule pileux ayant pour conséquence finale la perte définitive partielle ou générale des cheveux.

Dans un nombre important de cas, la chute précoce des cheveux survient chez des sujets prédisposés génétiquement et elle atteint notamment les hommes. II s'agit plus particulièrement de l'alopécie androgénétique ou androgénique ou encore androgéno-génétique.

On recherche depuis de nombreuses années, dans l'industrie cosmétique ou pharmaceutique, des substances permettant de supprimer ou de réduire l'alopécie, et notamment d'induire ou de stimuler la croissance des cheveux ou de diminuer leur chute.

Dans cette optique, on a certes déjà proposé un grand nombre de composés actifs très divers, comme par exemple le 2,4-diamino 6-pipéridino pyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Il reste, d'une manière générale, qu'il serait intéressant et utile de pouvoir disposer de composés actifs autres que ceux déjà connus, potentiellement plus actifs et/ou moins toxiques.

Ce but et d'autres sont atteints par la présente invention qui concerne des composés répondant à la formule générale (I) : 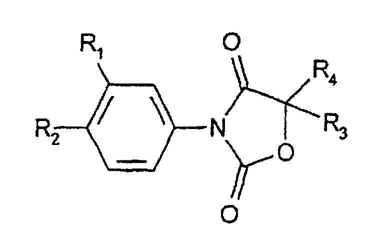 dans laquelle
R1 est un atome d'halogène ou un groupement cyano ou un groupement alkyle, en C₁ - C₄ substitué par au moins un atome d'halogène ;
R2 est un groupement cyano ou un atome d'halogène ou un carboxylate d'alkyle en C₁ - C₄ ;
R3 est un atome d'hydrogène ou un groupement alkyle, en C₁ - C₄, éventuellement substitué par au moins un atome d'halogène ;
R4 est un groupement alkyle en C₁ - C₄, substitué par au moins un atome d'halogène.

L'invention conceme également les isomères optiques, seuls ou en mélange en toutes proportions, les formes acylées ou encore les sels pharmaceutiquement acceptables de ces composés.

Le document FR 2275206 décrit le 3-(3', 4'-dichlorophényl) 5,5-diméthyl oxazolidine 2,4-dione pour ces propriétés anti-androgènes et son utilisation à titre de médicament.
Le document DE-A-3115650 décrit un procédé de préparation des 2,4 oxazolidine-diones substituées.
Le document FR 1284516 décrit l'utilisation de 2,4 oxazolidine-diones substituées comme agents susceptibles d'agir sur la croissance des plantes.
Aucun de ces documents ne décrit les composés de la présente demande.

Par atomes d'halogène on entend de préférence selon l'invention les atomes de fluor, de chlore ou de brome.

Par groupement alkyle en C₁ - C₄ on entend de préférence selon l'invention les radicaux alkyles linéaires ou ramifiés en C₁ - C₄ et en particulier les radicaux méthyle, éthyle, propyle, isopropyle, butyle, isobutyle ou tertiobutyle et plus particulièrement le radical méthyle.

Par radical alkyle substitué par au moins un atome d'halogène on entend de préférence les radicaux définis ci-dessus pour lesquels au moins un atome d'hydrogène est substitué par un atome d'halogène, y compris les radicaux perhalogénés pour lesquels tous les atomes d'hydrogène sont remplacés par autant d'atomes d'halogènes.

D'une manière préférentielle les radicaux alkyles substitués par au moins un atome d'halogène sont substitués par au moins un atome de fluor. Les radicaux perhalogénés sont préférentiellement des radicaux perfluorés, en particulier des radicaux perfluorométhyles.

Selon une forme de réalisation particulière de l'invention, R1 est préférentiellement un atome de chlore.

Selon une autre forme de réalisation de l'invention, R1 est un radical méthyle perfluoré.

Lorsque R2 est un atome d'halogène, R2 est préférentiellement un atome de chlore
De manière préférentielle R3 est un radical méthyle perfluoré.

Avantageusement, R4 est un radical méthyle substitué par au moins un atome d'halogène de préférence un radical méthyle perfluoré.

R3 et R4 peuvent être identiques mais de préférence R3 et R4 ont une signification différente.

On peut citer comme composés de formule (I):
le 4-(5-méthyl-2,4-dioxo-5-trifluorométhyl)-oxazolidin-3-yl)-2-trifluorométhylbenzanitrile,
le 4-(5-méthyl-2,4-dioxo-5-phényl)-oxazolidin-3-yl)-2-trifluorométhylbenzonitrile,
le 4-(5-méthyl-2,4-dioxo-5-trifluorométhyl)-oxazolidin-3-yl)-2-cyanobenzonitrile,
l'ester éthylique de l'acide 4-(5-méthyl-2,4-dioxo-5-trifluoromethyl)-oxazolidin-3-yl)-2-cyanobenzoïque

Parmi ces composés on préfère tout particulièrement :
le 4-(5-méthyl-2,4-dioxo-5-trifluorométhyl)-oxazolidin-3-yl)-2-trifluorométhylbenzonitrile.

Un deuxième objet de l'invention est un procédé de préparation des composés de formule (I) tels que définis ci-dessus.

Ce procédé est caractérisé par le fait que l'on fait réagir dans un solvant anhydre approprié en présence d'une amine, un isocyanate substitué en 3, 4 de formule générale : 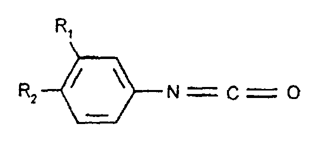 et une cyanydrine de formule générale 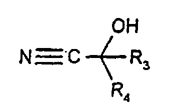 pour lesquelles R1, R2, R3 et R4 ont les définitions précédentes.

Comme solvant anhydre on peut utiliser le tétrahydrofuranne (THF) ou le toluène

Comme amine on peut utiliser la triéthylènediamine, la triéthylamine, la 4-méthylmorpholine ou encore de la pyridine.

Le mélange obtenu est ensuite traité en milieu acide en présence d'alcool, afin d'éliminer le plus de résidus isocyanates restants. Ce milieu acide peut être constitué par exemple d'un mélange d'acide chlorhydrique et de méthanol.

Le dérivé aryl 2,4-dioxo oxazolidine formé est extrait à l'aide d'un solvant organique comme le dichlorométhane ou l'acétate d'éthyle, puis séché et enfin purifié par chromatographie sur colonne de silice (Patton, T.L. J. Org. Chem. (1967) 32(2), 383-388).

Un exemple de préparation des composés selon l'invention est donné par ailleurs dans les exemples.

Un troisième objet de l'invention conceme des compositions cosmétiques ou pharmaceutiques, particulièrement dermatologiques, qui comprennent au moins un des composés répondant à la formule (I) définis ci-dessus.

Bien entendu les compositions selon l'invention peuvent comprendre les composés de formule (I) seuls ou en mélanges en toutes proportions.

La quantité de composés de formule (I) contenue dans les compositions de l'invention est bien entendu fonction de l'efet recherché et peut donc varier dans une large mesure.

Pour donner un ordre de grandeur, si la composition est une composition cosmétique, elle peut contenir au moins un composé de formule (I) en une quantité représentant de 0,001% à 10% du poids total de la composition et préférentiellement en une quantité représentant de 0,01% à 5% du poids total de la composition.

Pour donner un ordre de grandeur, si la composition est une composition pharmaceutique, elle peut contenir au moins un composé de formule (I) en une quantité représentant de 0,005% à 20% du poids total de la composition et préférentiellement en une quantité représentant de 0,05% à 10% du poids total de la composition.

La composition peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.
La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions selon l'invention peuvent également consister en des préparations solides constituant des savons ou des pains de nettoyage.

Les compositions peuvent aussi être conditionnées sous forme d'une composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosméticue peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, potyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins composé de formule (I) à d'autres agents actifs. Parmi ces agents actifs, on peut citer à titre d'exemple :
- les agents améliorant l'activité sur la repousse et/ou sur le freinage de la chute des cheveux, et ayant déjà été décrits pour cette activité comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle, les dérivés de pyrimidine, comme le 2,4-diamino 6-piperidinopyrimidine 3-oxyde ou "Minoxidil" décrit dans les brevets US 4 139 619 et US 4 596 812, les agents favorisant la repousse des cheveux comme ceux décrits par la demanderesse dans la demande de brevet européen publiée sous le numéro 0648488 ;
- les agents diminuant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens comme par exemple l'ibuprofène et ses sels, le diclofénac et ses sels, l'acide acétylsalicylique, l'acétaminophène ou l'acide glycyrrhizique;
- les agents anesthésiques tels que le chlorhydrate de lidocaïne et ses dérivés ;
- les agents antiprurigineux comme la thénaldine, la triméprazine ou la cyproheptadine;
- les agents kératolytiques tels que les acides α- et β-hydroxycarboxyliques ou β-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'α-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle;
- les extraits d'origine végétale ou bactérienne.

A la liste ci-dessus, d'autres composés peuvent également être rajoutés, à savoir par exemple le Diazoxyde, la Spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoides, les acides eicosatétrayénoïque et eicosatriyénoïque ou leurs esters et amides, la vitamine D et ses dérivés, des extraits d'origine végétale ou bactérienne.
Ainsi, selon un mode particulier, la composition selon l'invention comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

On peut également envisager que la composition comprenant au moins un composé tel que défini précédement soit sous forme liposomée, telle que notamment décrite dans la demande de brevet WO 94/22468 déposée le 13 octobre 1994 par la société Anti Cancer Inc. Ainsi, le composé encapsulé dans les liposomes peut être délivré sélectivement au niveau du follicule pileux.

La composition pharmaceutique selon l'invention peut être administrée par voie parentérale, entérale ou encore par voie topique. De préférence, la composition pharmaceutique est administrée par voie topique.

Un quatrième objet de l'invention concerne l'utilisation à titre de principe actif, dans un milieu physiologiquement acceptable, dans une composition cosmétique ou pour la préparation d'une composition pharmaceutique, d'une quantité efficace d'au moins un composé de formule (I) définis ci-dessus.

Les composés de formule (I) sont en effet d'excellents ouvreurs de canaux potassiques, propriété principale du Minoxidil, seul composé reconnu à ce jour comme efficace dans les traitements de la chute des cheveux.

Ils sont également d'excellents antagonistes réceptoriels des androgènes, les androgènes étant responsables d'une forme particulièrement répandue de l'alopécie, l'alopécie androgéno-dépendante. Mais, on sait également que les androgènes sont impliqués dans l'hyperséborrhée et l'acné.

Ainsi, les composés de formule (I) présentent des activités remarquables qui justifient leur utilisation à titre de médicament, en particulier pour induire et/ou stimuler la croissance des cheveux et/ou freiner leur chute et/ou dans le traitement de l'hyperséborrhée et/ou de l'acné.

A la connaissance de la demanderesse il n'a jamais été proposé dans l'art antérieur l'utilisation de tels composés de la famille des aryl 2,4 dioxo oxazolidines à activité mixte, ouvreurs de canaux potassiques / anti-androgènes, pour lutter contre la chute des cheveux et/ou dans le traitement de l'hyperséborrhée et/ou de l'acné.

Ainsi, l'invention à pour objet l'utilisation à titre de principe actif, dans un milieu physiologiquement acceptable, dans une composition cosmétique ou pour la préparation d'une composition pharmaceutique, d'une quantité efficace d'au moins un composé de formule (I), ce composé ou les compositions étant destinés à induire et/ou stimuler la croissance des cheveux et/ou freiner leur chute et/ou à traiter l'hyperséborrhée et/ou l'acné.

Préférentiellement, le composé utilisé selon l'invention est choisi parmi
le 4-(5-méthyl-2,4-dioxo-5-trifluorométhyl)-oxazolidin-3-yl)-2-trifluorométhylbenzonitrile,
le 4-(5-méthyl-2,4-dioxo-5-phényl)-oxazolidin-3-yl)-2-trifluorométhylbenzonitrile,
le 4-(5-méthyl-2,4-dioxo-5-trifluorométhyl)-oxazolidin-3-yl)-2-cyanobenzonitrile,
l'ester éthylique de l'acide 4-(5-méthyl-2,4-dioxo-5-trifluorométhyl)-oxazolidin-3-yl)-2-cyanobenzoïque

Parmi ces composés on préfère tout particulièrement :
le 4-(5-méthyl-2,4-dioxo-5-trifluorométhyl)-oxazolidin-3-yl)-2-trifluorométhylbenzonitrile.

Bien entendu les composés peuvent être utilisés seuls ou en mélange.

La composition cosmétique selon l'invention est à appliquer sur les zones alopéciques du cuir chevelu et des cheveux d'un individu, et est éventuellement laissée en contact plusieurs heures et est éventuellement à rincer. On peut, par exemple, appliquer la composition contenant une quantité efficace d'au moins un composé tel que défini précédement, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des cheveux et/ou du cuir chevelu et/ou la peau, caractérisé par le fait qu'il consiste à appliquer sur les cheveux et/ou le cuir chevelu et/ou la peau, une composition cosmétique comprenant une quantité efficace d'au moins composé tel que défini précédement, à laisser celle-ci en contact avec les cheveux et/ou le cuir chevelu et/ou la peau, et éventuellement à rincer.

Le procédé de traitement présente les caractéristiques d'un procédé cosmétique dans la mesure où il permet d'améliorer l'esthétique des cheveux et/ou de la peau en leur donnant une plus grande vigueur et un aspect amélioré.

On va maintenant donner à titre d'illustration des exemples qui ne sauraient limiter en aucune façon la portée de l'invention.

### Exemple 1 : Synthèse du 4-(5-méthyl-2,4-dioxo-5-trifluorométhyl)-oxazolidin-3-yl)-2-trifluorométhylbenzonitrile

On dissout 1.09 g de 4-isocyanato 2-trifluorométhyl benzonitrile dans 20 ml de THF anhydre. On additionne 150 µl de Triéthylamine puis 860 mg de trifluoroacétone cyanohydrine en solution dans 10 ml de THF anhydre. Le milieu réactionnel est agité 2h à température ambiante.

On additionne 5 ml de méthanol puis après 30 mn, 10 ml d'acide chlorhydrique 1N: Après 2 h à température ambiante, on ajoute 20 ml d'eau. Le milieu réactionnel est extrait par 4 x 25 ml de dichlorométhane. La phase organique est lavée par 25 ml d'eau, séchée sur sulfate de sodium, évaporée à sec. Le résidu obtenu est purifié par chromatographie sur colonne de silice, l'éluant étant du dichlorométhane. On obtient 540 mg d'un précipité blanc.

### Analyses

* Spectre RMN 500 MHz dans CDCl₃ : conforme à la structure

| * Analyse élémentaire : | | C | H | N | F |
|---|---|---|---|---|---|
| | % calc | 44.32 | 1.70 | 7.95 | 32.38 |
| | %Tr | 44.52 | 1.76 | 8.00 | 32.53/32.24 |

* Point de fusion : Kofler = 90-92°C

### Exemple 2 : Mesure de l'affinité du 4-(5-méthyl-2,4-dioxo-5-trifluorométhyl)-oxazolidin-3-yl)-2-trifluorométhylbenzonitrile pour le récepteur aux androgènes.

Ces mesures d'affinité réceptorielle pour le récepteur aux androgènes sont effectuées selon la méthode de Schilling et Liao, décrite dans "The Prostate", 1984, 5, p. 581-588.

Le composé est testé à plusieurs concentrations. Au cours de l'expérimentation, la molécule de référence (mibolerone) est parallèlement testée à 8 concentrations afin de valider l'expérience.

| Composé à la concentration de : | 0,1 µM | 1 µM | 10 µM |
|---|---|---|---|
| 4-(5-méthyl-2,4-dioxo-5-trifluorométhyl)oxazolidin-3-yl)-2-trifluorométhylbenzonitrile | 2 | 17 | 56 |

Les résultats sont exprimés en % d'inhibition ce la fixation de la testostérone sur son récepteur. La référence interne (mibolerone) inhibe de 50 % cette fixation à une concentration de 4,3 nM.

### Exemple 3 :

Mesure de l'activité de type "ouvreur de canaux potassiques" du 4-(5-méthyl-2,4-dioxo-5-trifluorométhyl)-oxazolidin-3-yl)-2-trifluorométhylbenzonitrile par la mesure *in vitro* du pouvoir relaxant des molécules sur des anneaux d'aorte thoracique.

Les expériences sont réalisées selon les méthodes de Newgreen et al (Br. J. Pharmacol., 100, 1990, p. 605-613), Bray et al (Arch. Pharmacol., 344, 1991, p. 351-359) et Wickerden et al (Br. J. Pharmacol., 103, 1991, 1148-1152).

Après leur installation dans les cuves à organes isolés, les tissus (muscles lisses aortiques) sont soumis à une tension initiale de 2 g.

Après une période d'équilibration, les tissus sont exposés à une solution de chlorure de potassium (KCI, à 20 mM) pour obtenir une réponse contractile soutenue.
Après stabilisation de cette réponse contractile, l'activité relaxante (de type ouvreur de canaux potassiques) des molécules à tester est évaluée en dose-réponses.

Au cours de l'expérimentation, deux molécules références sont utilisées : la cromakalim et le Minoxidil.

| Composé à la concentration de : | 0,1 µM | 1 µM | 10 µM |
|---|---|---|---|
| 4-(5-méthyl-2,4-dioxo-5-trifluorométhyl)oxazolidin-3-yl)-2-trifluorométhylbenzonitrile | 12 | 69 | 95 |
| Minoxidil | 25 | 65 | 95 |
| Cromakalim | 46 | 86 | 100 |

Résultats exprimés en % d'inhibition de la contraction provoquée par le KCI à 20mM.

Les résultats montrent que le 4-(5-méthyl-2,4-dioxo-5-trifluorométhyl)-oxazolidin-3-yl)-2-trifluorométhylbenzonitrile présente une activité mixte, avec une activité préférentielle pour la facette K⁺ Agoniste.

### Exemple 4: Exemples de compositions contenant un aryl 2,4 dioxo oxazolidine.

Ces compositions sont obtenues par les techniques habituelles couramment utilisées en cosmétique ou en pharmacie.

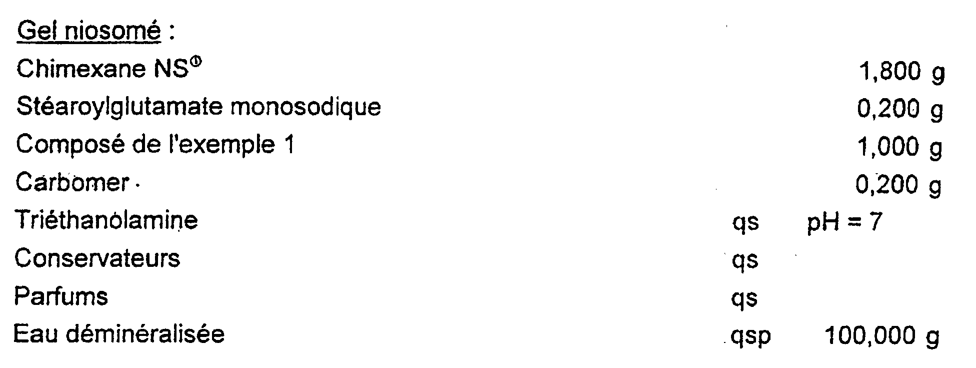

On applique ce gel sur le cuir chevelu, une à deux fois par jour.

| Lotion antichute : | | |
|---|---|---|
| Composé de l'exemple 1 | | 2,000 g |
| Propylène glycol | | 30,000 g |
| Alcool éthylique | | 40,500 g |
| Eau | qsp | 100,000 g |

On applique cette lotion sur le cuir chevelu, une à deux fois par jour, à raison d'1 ml par application.

| Lotion antichute épaissie : | | |
|---|---|---|
| Composé de l'exemple 1 | | 0,500 g |
| Kawaïne | | 2,000 g |
| Hydroxypropylcellulose (Klucel G de la société Hercules) | | 3,500 g |
| Alcool éthylique | qsp | 100,000 g |

On applique cette lotion épaissie sur le cuir chevelu, une à deux fois par jour, à raison d'1 ml par application.

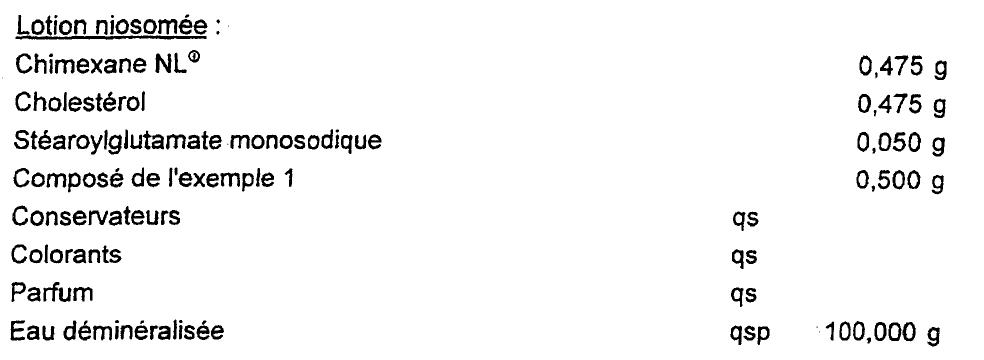

On applique cette lotion sur le cuir chevelu, une à deux fois par jour, à raison d'1 ml par application.

| lotion antichute : | | |
|---|---|---|
| Composé de l'exemple 1 | | 0,050 g |
| Monométhyléther de propylèneglycol (Dowanol PM de Dow Chemical) | | 20,000 g |
| Hydroxypropylcellulose (Klucel G de la société Hercules) | | 3,000 g |
| Alcool éthylique | | 40,000 g |
| Minoxidil | | 2,000 g |
| Eau | qsp | 100,000 g |

On applique cette lotion épaissie sur le cuir chevelu, une à deux fois par jour, à raison d'1 ml par application.

| Lotion antichute : | | |
|---|---|---|
| Composé de l'exemple 1 | | 2,000 g |
| Propylène glycol | | 10,000 g |
| Alcool isopropylique | qsp | 100,000 g |

On applique 1 ml de cette lotion sur le cuir chevelu, à la fréquence de une à deux fois par jour.

Avec chacune des compositions décrites dans les exemples ci-dessus, on a constaté, après plusieurs mois de traitement et selon les sujets traités, un ralentissement de la chute des cheveux et/ou un effet repousse.

## Revendications

1. Un composé répondant à la formule générale (I): 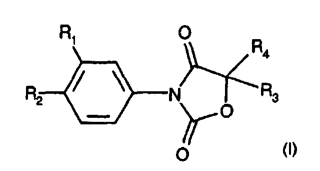 dans laquelle
R1 est un atome d'halogène ou un groupement cyano ou un groupement alkyle, en C₁ - C₄, substitué par au moins un atome d'halogène;
R2 est un groupement cyano ou un atome d'halogène, ou un carboxylate d'alkyle en C₁ - C₄;
R3 est un atome d'hydrogène ou un groupement alkyle, en C₁ - C₄, éventuellement substitué par au moins un atome d'halogène ;
R4 est un groupement alkyle, en C₁ - C₄, substitué par au moins un atome d'halogène ;
ou ses isomères optiques, seuls ou en mélange en toutes proportions, ou encore ses sels pharmaceutiquement acceptables.

2. Composé selon la revendication précédente, **caractérisé par le fait que** R1 est un atome de chlore.

3. Composé selon la revendication 1, **caractérisé par le fait que** R1 est un est un radical méthyle perfluoré.

4. Composé selon l'une quelconque des revendications 1 à 3, **caractérisé par le fait que** R2 est un atome de fluor.

5. Composé selon l'une quelconque des revendications 1 à 4, **caractérisé par le fait que** R3 est un radical méthyle perfluoré.

6. Composé selon l'une quelconque des revendications 1 à 5, **caractérisé par le fait que** R4 est un radical méthyle substitué par au moins un atome d'halogène de préférence un radical méthyle perfluoré.

7. Composé selon l'une quelconque des revendications 1 à 7, **caractérisé par le fait que** le composé est choisi parmi :
le 4-(5-méthyl-2,4-dioxo-5-trifluorométhyl)-oxazolidin-3-yl)-2-trifluoromothylbenzonitrile,
le 4-(5-méthyl-2,4-dioxo-5-phényl)-oxazolidin-3-yl)-2-trifluorométhylbenzonitrile,
le 4-(5-méthyl-2,4-dioxo-5-trifluorométhyl)-oxazolidin-3-yl)-2-cyanobenzonitrile,
l'ester éthylique de l'acide 4-(5-méthyl-2,4-dioxo-5-trifluorométhyl)-oxazolidin-3-yl)-2-cyanobenzoïque.

8. Procédé de préparation d'un composé de formule (I) tel que défini selon l'une quelconque des revendications 1 à 7, **caractérisé par le fait que** l'on fait réagir dans un solvant anhydre approprié, un isocyanate substitué en 3, 4 de formule générale : 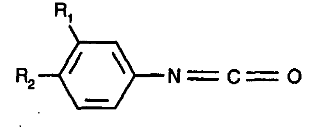 et une cyanydrine de formule générale 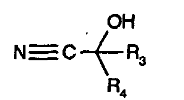 pour lesquelles R1, R2, R3 et R4 ont les définitions précédentes,
en présence d'une amine, que le mélange obtenu est ensuite traité par un acide en présence d'alcool et que le dérivé aryl 2,4-dioxo oxazolidine formé est extrait à l'aide d'un solvant organique, séché puis purifié par chromatographie sur colonne de silice.

9. Procédé selon la revendication 8, **caractérisé par le fait que** le solvant anhydre est du tétrahydrofuranne.

10. Procédé selon l'une des revendications 8 ou 9, **caractérisé par le fait que** l'amine est la triéthylamine.

11. Procédé selon l'une des revendications 8 à 10, **caractérisé par le fait que** le mélange obtenu est traité par un mélange d'acide chlorhydrique et de méthanol.

12. Procédé selon l'une des revendications 8 à 11, **caractérisé par le fait que** le solvant organique est du dichlorométhane.

13. Composition comprenant au moins un composé de formule (I) tel que défini selon l'une quelconque des revendications 1 à 7.

14. Composition selon la revendication 13, **caractérisée par le fait qu'**elle est destinée à un usage cosmétique ou pharmaceutique.

15. Composition cosmétique selon l'une quelconque des revendications 13 ou 14, **caractérisée par le fait qu'**elle comprend au moins un composé de formule (I) en une quantité représentant de 0,001% à 10% du poids total de la composition et préférentiellement en une quantité représentant de 0,01% à 5% du poids total de la composition.

16. Composition pharmaceutique selon l'une quelconque des revendications 13 ou 14, **caractérisée par le fait qu'**elle comprend au moins un composé de formule (I) en une quantité représentant de 0,005% à 20% du poids total de la composition et préférentiellement en une quantité représentant de 0,05% à 10% du poids-total de la composition.

17. Composition selon l'une quelconque des revendications 13 à 16, **caractérisée par le fait qu'**elle comprend également au moins un agent choisi parmi les agents antibactériens, les antiparasitaires, les antifongiques, les antiviraux, les anti-inflammatoires, les antiprurigineux, les anesthésiques, les kératolytiques, les anti-radicaux libres, les anti-séborrhéiques, les antipelliculaires, les antiacnéiques et/ou les agents diminuant la différenciation et/ou la prolifération et/ou la pigmentation cutanée, les extraits d'origine végétale ou bactérienne.

18. Composition selon l'une quelconque des revendications 13 à 17, **caractérisée par le fait qu'**elle est destinée à induire et/ou stimuler la croissance des cheveux et/ou freiner leur chute et/ou à traiter l'hyperséborrhée et/ou l'acné.

19. A titre de médicaments les composés de formules générale (I) 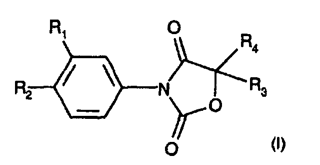 tels que définis dans les revendications 1 à 7.

20. Utilisation dans une composition cosmétique ou pour la préparation d'une composition pharmaceutique, d'une quantité efficace d'au moins un composé répondant à la formule générale (I) tel que défini selon l'une quelconque des revendications 1 à 7, ce composé ou cette composition étant destiné à induire et/ou stimuler la croissance des cheveux et/ou freiner leur chute et/ou traiter l'hypersébhorrée et/ou l'acné.

21. Procédé de traitement cosmétique des cheveux et/ou du cuir chevelu, **caractérisé par le fait qu'**il consiste à appliquer sur les cheveux et/ou le cuir chevelu et/ou sur la peau, une composition cosmétique telle que définie dans l'une quelconque des revendications 13 à 15 ou 17 ou 18, à laisser celle-ci en contact avec les cheveux et/ou le cuir chevelu et/ou la peau, et éventuellement à rincer.

## Claims

1. Compound corresponding to the general formula (I): 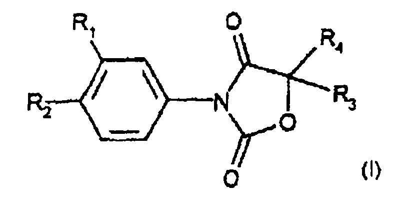 in which
R1 is a halogen atom or a cyano group or a C₁-C₄ alkyl group substituted with at least one halogen atom;
R2 is a cyano group or a halogen atom or a C₁-C₄ alkyl carboxylate;
R3 is a hydrogen atom or a C₁-C₄ alkyl group optionally substituted with at least one halogen atom;
R4 is a C₁-C₄ alkyl group substituted with at least one halogen atom;
or optical isomers thereof, alone or mixed in all proportions, or pharmaceutically acceptable salts thereof.

2. Compound according to the preceding claim, **characterized in that** R1 is a chlorine atom.

3. Compound according to Claim 1, **characterized in that** R1 is a perfluoromethyl radical.

4. Compound according to any one of Claims 1 to 3, **characterized in that** R2 is a fluorine atom.

5. Compound according to any one of Claims 1 to 4, **characterized in that** R3 is a perfluoromethyl radical.

6. Compound according to any one of Claims 1 to 5, **characterized in that** R4 is a methyl radical substituted with at least one halogen atom, preferably a perfluoromethyl radical.

7. Compound according to any one of Claims 1 to 7, **characterized in that** the compound is chosen from:
4-(5-methyl-2,4-dioxo-5-trifluoromethyl)-3-oxazolidinyl)-2-trifluoromethylbenzonitrile,
4-(5-methyl-2,4-dioxo-5-phenyl)-3-oxazolidinyl)-2-trifluoromethylbenzonitrile,
4-(5-methyl-2,4-dioxo-5-trifluoromethyl)-3-oxazolidinyl)-2-cyanobenzonitrile,
ethyl 4-(5-methyl-2,4-dioxo-5-trifluoromethyl)-3-oxazolidinyl)-2-cyanobenzoate.

8. Process for preparing a compound of formula (I) as defined according to any one of Claims 1 to 7, **characterized in that** a 3,4-substituted isocyanate of general formula: 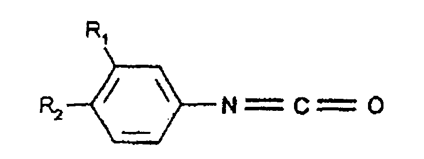 and a cyanohydrin of general formula 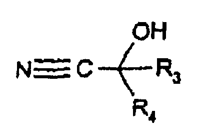 for which R1, R2, R3 and R4 have the above definitions,
are reacted in a suitable anhydrous solvent, in the presence of an amine, the mixture obtained is then treated with an acid in the presence of alcohol and the aryl 2,4-dioxo oxazolidine derivative formed is extracted using an organic solvent, dried and then purified by chromatography on a column of silica.

9. Process according to Claim 8, **characterized in that** the anhydrous solvent is tetrahydrofuran.

10. Process according to either of Claims 8 and 9, **characterized in that** the amine is triethylamine.

11. Process according to one of Claims 8 to 10, **characterized in that** the mixture obtained is treated with a mixture of hydrochloric acid and methanol.

12. Process according to one of Claims 8 to 11, **characterized in that** the organic solvent is dichloromethane.

13. Composition comprising at least one compound of formula (I) as defined according to any one of Claims 1 to 7.

14. Composition according to Claim 13, **characterized in that** it is intended for cosmetic or pharmaceutical use.

15. Cosmetic composition according to either of Claims 13 and 14, **characterized in that** it comprises at least one compound of formula (I) in an amount representing from 0.001% to 10% of the total weight of the composition and preferably in an amount representing from 0.01% to 5% of the total weight of the composition.

16. Pharmaceutical composition according to either of Claims 13 and 14, **characterized in that** it comprises at least one compound of formula (I) in an amount representing from 0.005% to 20% of the total weight of the composition and preferably in an amount representing from 0.05% to 10% of the total weight of the composition.

17. Composition according to any one of Claims 13 to 16, **characterized in that** it also comprises at least one agent chosen from antibacterial agents, antiparasitic agents, antifungal agents, antiviral agents, anti-inflammatories, anti-pruriginous agents, anaesthetics, keratolytic agents, free-radical scavengers, anti-seborrhoeic agents, antidandruff agents, antiacne agents and/or agents for reducing skin differentiation and/or proliferation and/or pigmentation, and extracts of plant or bacterial origin.

18. Composition according to any one of Claims 13 to 17, **characterized in that** it is intended to induce and/or stimulate hair growth and/or to stop hair loss and/or to treat hyperseborrhoea and/or acne.

19. As medicinal products, the compounds of general formula (I) 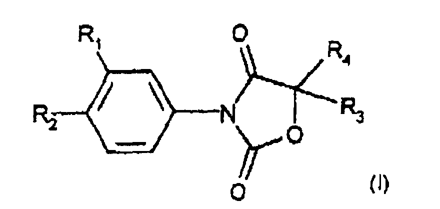 as defined in Claims 1 to 7.

20. Use, in a cosmetic composition or for the preparation of a pharmaceutical composition, of an effective amount of at least one compound corresponding to the general formula (I) as defined according to any one of Claims 1 to 7, this compound or this composition being intended to induce and/or stimulate hair growth and/or to stop hair loss and/or to treat hyperseborrhoea and/or acne.

21. Cosmetic treatment process for the hair and/or the scalp, **characterized in that** it consists in applying to the hair and/or the scalp and/or the skin a cosmetic composition as defined in any one of Claims 13 to 15 or 17 or 18, in leaving the said composition in contact with the hair and/or the scalp and/or the skin, and in optionally rinsing it off.

## Patentansprüche

1. Verbindungen der allgemeinen Formel (I): 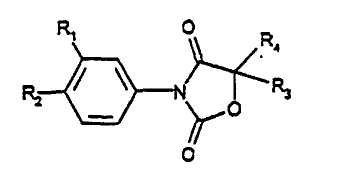 worin bedeuten:
R₁ Halogen, Cyano oder eine C₁₋₄-Alkylgruppe, die mit mindestens einem Halogenatom substituiert ist;
R₂ Cyano, Halogen oder eine C₁₋₄-Alkylcarboxylatgruppe;
R₃ Wasserstoff oder eine C₁₋₄-Alkylgruppe, die gegebenenfalls mit mindestens einem Halogenatom substituiert ist;
R₄ eine C₁₋₄-Alkylgruppe, die mit mindestens einem Halogenatom substituiert ist;
oder ihre optischen Isomere einzeln oder im Gemisch in beliebigen Mengenanteilen, oder die pharmazeutisch akzeptablen Salze dieser Verbindungen.

2. Verbindungen nach dem vorhergehenden Anspruch, **dadurch gekennzeichnet, daß** R₁ Chlor bedeutet.

3. Verbindungen nach Anspruch 1, **dadurch gekennzeichnet, daß** R₁ die perfluorierte Methylgruppe ist.

4. Verbindungen nach einem der Ansprüche 1 bis 3, **dadurch gekennzeichnet, daß** R₂ Fluor bedeutet.

5. Verbindungen nach einem der Ansprüche 1 bis 4, **dadurch gekennzeichnet, daß** R₃ die perfluorierte Methylgruppe bedeutet.

6. Verbindungen nach einem der Ansprüche 1 bis 5, **dadurch gekennzeichnet, daß** R₄ eine Methylgruppe ist, die mit mindestens einem Halogenatom substituiert ist, vorzugsweise die perfluorierte Methylgruppe.

7. Verbindungen nach einem der Ansprüche 1 bis 7, **dadurch gekennzeichnet, daß** sie ausgewählt sind unter:
- 4-(5-Methyl-2,4-dioxo-5-trifluormethyl)-oxazolidin-3-yl)-2-trifluormethylbenzonitril;
- 4-(5-Methyl-2,4-dioxo-5-phenyl)-oxazolidin-3-yl)-2-trifluormethylbenzonitril;
- 4-(5-Methyl-2,4-dioxo-5-trifluormethyl)-oxazolidin-3-yl)-2-cyanobenzonitril;
- dem Ethylester von 4-(5-Methyl-2,4-dioxo-5-trifluormethyl)-oxazolidin-3-yl)-2-cyanobenzoesäure.

8. Verfahren zur Herstellung einer Verbindung der Formel (I) nach einem der Ansprüche 1 bis 7, **dadurch gekennzeichnet, daß** in einem geeigneten wasserfreien Lösungsmittel in Gegenwart eines Amins ein in 3,4-Stellung substituiertes Isocyanat der folgenden allgemeinen Formel: 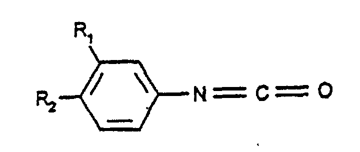 mit einem Cyanohydrin der folgenden allgemeinen Formel umgesetzt wird: 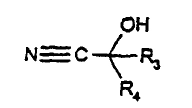 wobei die Gruppen R₁, R₂, R₃ und R₄ die oben angegebenen Bedeutungen aufweisen,
und das erhaltene Gemisch in Gegenwart eines Alkohols mit einem sauren Medium versetzt und das gebildete Aryl-2,4-dioxooxazolidinderivat wird mit einem organischen Lösungsmittel extrahiert, getrocknet und chromatographisch an einer Kieselgelsäule gereinigt wird.

9. Verfahren nach Anspruch 8, **dadurch gekennzeichnet, daß** das wasserfreie Lösungsmittel das Tetrahydrofuran ist.

10. Verfahren nach einem der Ansprüche 8 oder 9, **dadurch gekennzeichnet, daß** das Amin das Triethylamin ist.

11. Verfahren nach einem der Ansprüche 8 bis 10, **dadurch gekennzeichnet, daß** das erhaltenen Gemisch mit einem Gemisch von Salzsäure und Methanol versetzt wird.

12. Verfahren nach einem der Ansprüche 8 bis 11, **dadurch gekennzeichnet, daß** das organische Lösungsmittel das Dichlormethan ist.

13. Zusammensetzung, die mindestens eine Verbindung der Formel (I) nach einem der Ansprüche 1 bis 7 enthält.

14. Zusammensetzung nach Anspruch 13, **dadurch gekennzeichnet, daß** sie in der Kosmetik oder Pharmazie verwendet werden soll.

15. Kosmetische Zusammensetzung nach einem der Ansprüche 13 oder 14, **dadurch gekennzeichnet, daß** sie mindestens eine Verbindung der Formel (I) in einer Menge im Bereich von 0,001 bis 10 % des Gesamtgewichts der Zusammensetzung und vorzugsweise in einer Menge von 0,01 bis 5 % des Gesamtgewichts der Zusammensetzung enthält.

16. Pharmazeutische Zusammensetzung nach einem der Ansprüche 13 oder 14, **dadurch gekennzeichnet, daß** sie mindestens eine Verbindung der Formel (I) in einer Menge im Bereich von 0,005 bis 20 % des Gesamtgewichts der Zusammensetzung und vorzugsweise in einer Menge von 0,05 bis 10 % des Gesamtgewichts der Zusammensetzung enthält.

17. Zusammensetzung nach einem der Ansprüche 13 bis 16, **dadurch gekennzeichnet, daß** sie ferner mindestens einen Wirkstoff enthält, der unter den antibakteriellen Wirkstoffen, antiparasitären Wirkstoffen, Antimykotika, antiviralen Wirkstoffen, entzündungshemmenden Wirkstoffen, juckreizstillenden Mitteln, anästhesierenden Wirkstoffen, Keratolytika, Radikalfängern gegen freie Radikale, Antiseborrhöika, Antischuppenmitteln, Wirkstoffen gegen Akne und/oder Mitteln, die die Differenzierung und/oder Proliferation und/oder Pigmentierung der Haut vermindern, und Extrakten pflanzlicher oder bakterieller Herkunft ausgewählt ist.

18. Zusammensetzung nach einem der Ansprüche 13 bis 17, **dadurch gekennzeichnet, daß** sie zur Induzierung und/oder Stimulation des Haarwachstums und/oder zur Verlangsamung des Haarausfalls und/oder zur Behandlung von Hyperseborrhoe und/oder Akne dienen soll.

19. Verbindungen der allgemeinen Formel (I): 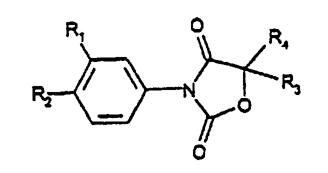 nach den Ansprüchen 1 bis 7 als Arzneimittel.

20. Verwendung einer wirksamen Menge mindestens einer Verbindung der Formel (I) nach einem der Ansprüche 1 bis 7 in einer kosmetischen Zusammensetzung oder zur Herstellung einer pharmazeutischen Zusammensetzung, wobei die Verbindung oder die Zusammensetzungen zur Induzierung und/oder Stimulation des Haarwachstums und/oder zur Verlangsamung des Haarausfalls und/oder zur Behandlung von Hyperseborrhoe und/oder Akne vorgesehen sind.

21. Verfahren zur kosmetischen Behandlung der Haare und/oder der Kopfhaut, **dadurch gekennzeichnet, daß** es darin besteht, daß auf die Haare und/oder die Kopfhaut und/oder die Haut eine kosmetische Zusammensetzung nach einem der Ansprüche 13 bis 15 oder 17 oder 18 aufgetragen wird, diese mit den Haaren und/oder der Kopfhaut und/oder der Haut in Kontakt belassen wird und gegebenenfalls gespült wird.
